# EUROPEAN PATENT SPECIFICATION

(11) **EP 0 888 132 B1**
(45) Date of publication and mention of the grant of the patent: **24.09.2008**
(21) Application number: 97902746.3
(22) Date of filing: 13.02.1997
(51) Int. Cl.: A61L 2/02, A61L 2/04

(54) **DEVICE FOR ELIMINATING CONTAMINANTS PRESENT IN A LIQUID**
GERÄT ZUR ELIMINIERUNG VON KRANKHEITSERREGERN IN EINER FLÜSSIGKEIT
DISPOSITIF POUR ELIMINER LES CONTAMINANTS PRESENTS DANS UN LIQUIDE

(30) Priority: 13.02.1996 NL 1002315; 13.02.1996 NL 1002316
(43) Date of publication of application: 07.01.1999
(73) Proprietor: Ball Packaging Europe GmbH, 40880 Ratingen (DE)
(72) Inventor: SAVRIJ DROSTE, Fred, Willem, NL-7231 DG Warnsveld (NL); TOENHAKE, Gerlof, Dinand, NL-6533 WN Nijmegen (NL); BRILMAN, Gerrit, Willem, NL-7437 CX Bathmen (NL)
(74) Representative: 't Jong, Bastiaan Jacob
(86) International application number: PCT/NL1997/000058
(87) International publication number: WO 1997/029786

(56) References cited:
- WO-A-96/09510
- FR-A- 1 219 346
- US-A- 4 620 962
- US-A- 4 759 848

## Description

A container for carbonated drinks can derive a part of its mechanical rigidity in the closed situation from the internal gas pressure. In analogous manner gas fillings are used for general application in for instance containers for foodstuffs wherein the internal pressure in a container is greater than the atmospheric pressure.

For use with foodstuffs such gases must display inert behaviour. Suitable for instance are the most common nitrogen, dinitrogen monoxide, argon, neon or other inert gases, carbon dioxide etc. Gases are also used to displace oxygen. The containers are not necessarily under pressure, although the gases can also be applied as propellant.

Devices are for instance known for admitting liquid nitrogen at a temperature of almost -200°C in droplet form into a container, for closing the container and for ensuring that, as a result of the subsequent natural slight heating, the liquid nitrogen passes into the gas phase and can develop gas pressure.

Suitable containers are metal cans, glasses or plastic pots with (screw) cover, pouches, bags, plastic trays with foil cover etc.

US-A-4 620 962 describes a device for eliminating micro organisms present in a liquid. Liquid nitrogen from a storage tank is heated in a heat exchanger and sterilized by ultraviolet sterilization or ultra filtration. This sterilized gas is then cooled down and stored in a second storage tank. This second storage tank is arranged in an isolation tank, which is filled with liquid nitrogen from the first storage tank. The sterilized nitrogen is discharged through a liquid nitrogen outlet. In order to avoid discharging of gaseous nitrogen, the liquid outlet is arranged at the bottom of the second storage tank. The liquid nitrogen is kept at a temperature of -195°C. The discharged sterilized nitrogen having a temperature of - 195°C has the disadvantage that by a slight temperature increase gas will be formed.

It is an object of the invention to provide a device with which the liquid nitrogen can be effectively rid of contaminants such as germs, bacteria and the like. Aseptic filling can then take place therewith.

In respect of the above the invention provides a device according to claim 1.

A high efficiency is obtained with an embodiment in which the heat exchanger is of the counterflow type.

The invention will now be elucidated with reference to the annexed drawings. In the drawings:
fig. 1 and 2 show schematically an embodiment of a filtering device according to the invention in two operating situations;
fig. 3 and 4 show schematically an example of a sterilizing device not according to the invention in two operating situations; and
fig. 5 is a schematic view of yet another example not according to the invention.

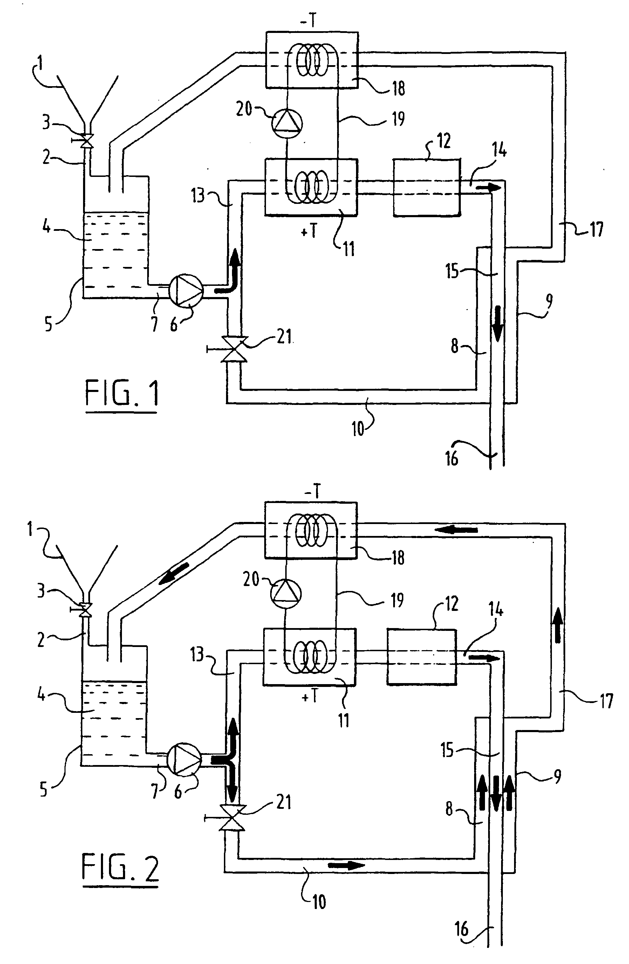

Insofar as applicable, corresponding components in fig. 1, 2, 3 and 4 are designated as far as possible with the same reference numerals.

Fig. 1 and 2 show schematically an embodiment of a filtering device according to the invention. This comprises a storage tank 5 to be filled with liquid nitrogen 4 at a temperature of about -200°C via a funnel 1 and a feed conduit 2 with valve 3, to which tank connects a feed conduit 7 provided with a pump 6. This conduit 7 branches into a cooling conduit 10 leading to the first circuit 8 of a heat exchanger 9 and a filter conduit 13 leading via a heating device 11 to a filter 12, the outlet 14 of which filter 12 leads to the second circuit 15 of heat exchanger 9 such that the heating device 11 heats the liquid nitrogen 4 to above its evaporation temperature into the gas phase and the gaseous nitrogen allowed through filter 12 condenses in heat exchanger 9. The outlet 16 of the second circuit 15 leads a flow of sterilized liquid nitrogen to an application station (not shown).

The outlet 17 of first circuit 8 is connected via a cooling device 18 to the storage tank 5. Recirculation of the liquid nitrogen used as cooling medium thus takes place.

As the figure shows, the heat exchanger 9 acts in counterflow.

The heating device 11 and the cooling device 18 form part of one system operating as heat pump which is designated schematically with 19 and comprises a pump 20.

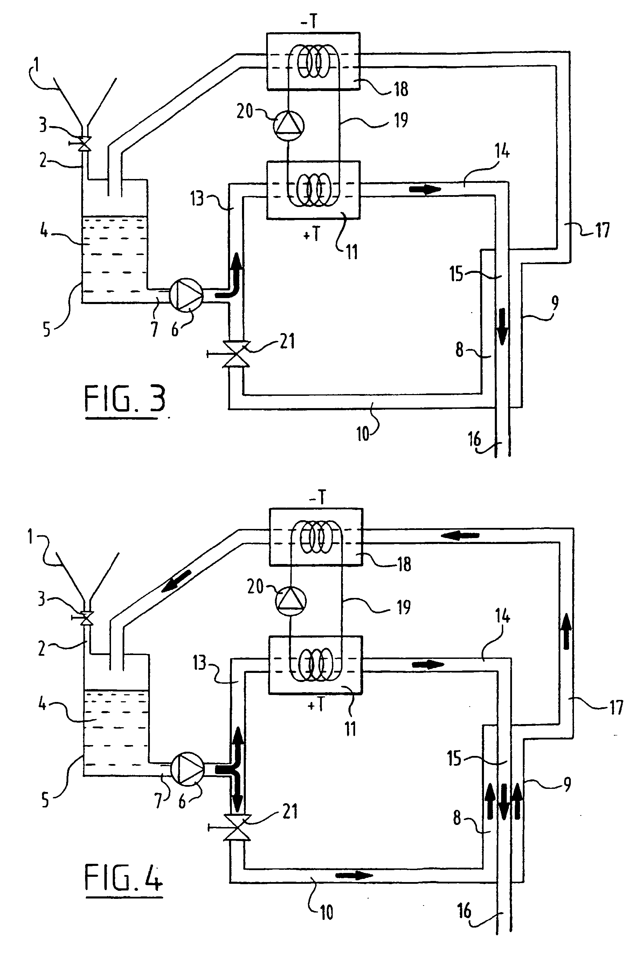

Fig. 3 and 4 shows schematically a sterilizing device. This comprises a storage tank 5 to be filled with liquid nitrogen 4 at a temperature of about -200°C via a funnel 1 and a feed conduit 2 with valve 3, to which tank connects a feed conduit 7 provided with a pump 6. This conduit 7 branches into a cooling conduit 10 leading to the first circuit 8 of a heat exchanger 9 and a filter conduit 13 leading via a heating device 11 to a device 12 (not shown) also for sterilizing, the outlet 14 of which device 12 leads to the second circuit 15 of heat exchanger 9 such that the heating device 11 heats the liquid nitrogen 4 to for instance about 150°C into the gas phase and the gaseous nitrogen allowed through device 12 condenses in heat exchanger 9. The outlet 16 of the second circuit 15 leads a flow of sterilized liquid nitrogen to an application station (not shown). The gas temperature in device 12 must always be sufficiently high to ensure that sterilization takes place effectively.

The outlet 17 of first circuit 8 is connected via a cooling device 18 to storage tank 5. Recirculation of the liquid nitrogen used as cooling medium thus takes place.

As the figure shows, the heat exchanger 9 acts in counterflow.

The heating device 11 and the cooling device 18 form part of one system operating as heat pump which is designated schematically with 19 and comprises a pump 20.

The valve 21 according to fig. 1, 2, 3 and 4 is arranged in cooling conduit 10 and can be placed in an open and closed position. In the situation of fig. 1 and 3 the valve 21 is closed, whereby all liquid pumped by pump 6 is supplied to conduit 13. In the situation of fig. 2 and 4 the valve 21 is opened, and a part of the liquid flow is allowed through conduit 13 and another part through conduit 10.

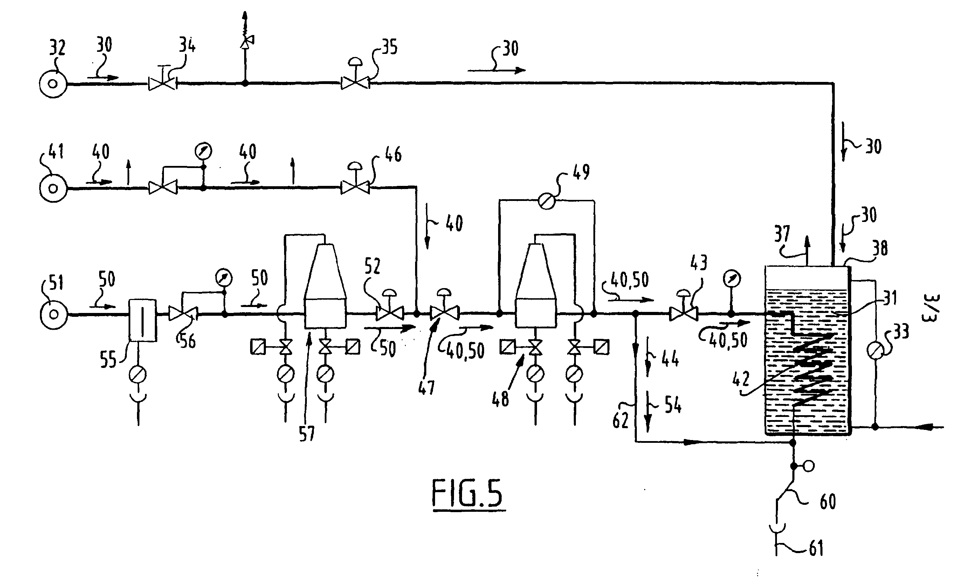

Fig. 5 shows a device not according to the invention in another example. Three circuits can be distinguished. The first circuit is designated with arrows 30 and serves to feed liquid nitrogen via feed 32 to a storage tank 31. A level meter 33 is added to the tank 31.

A second circuit is designated with arrows 40. This circuit is fed with gaseous nitrogen at room temperature and a pressure of several bar, for instance 2-6 bar. This nitrogen coming from feed 41 runs through heat exchanger spiral 42, is cooled by the liquid nitrogen 31 and leaves the device in liquid state via outlet 60 to be collected by a collecting station 61. By closing an aseptic control valve 43 the said medium flow through heat exchanger spiral 42 can be interrupted, whereby the gaseous nitrogen is fed directly via a bypass line 44 to conduit 60 for blowing out and drying thereof when this is necessary.

A third circuit is designated with arrows 50. At the inlet 51 thereof heated steam of for instance 130°C or heated nitrogen at a pressure in the order of 5-10 bar is supplied for sterilization purposes. By closing valve 46 and opening valve 52 the circuit 50 is effectively set into operation while switching off circuit 40. With the reverse operation the circuit 40 is effectively set into operation.

In the same manner as described for circuit 40 the discharge conduit 60 can also be sterilized via bypass line 62 by causing the heated steam or nitrogen to flow as according to arrow 54, not via heat exchanger spiral 42 (in the opened situation of valve 43) but via bypass line 62.

The reference numeral 55 designates a draining, 56 is a pressure regulator, 57 a steam filter with valves added thereto, 47 a control valve, 48 a sterile filter with valves added thereto, 49 a pressure difference sensor, 34 a control valve, 35 an on/off valve. Reference numeral 37 designates a discharge conduit for gaseous nitrogen on the top of storage tank 38. This conduit 37 can be connected to a cooling device which lowers the temperature of the gaseous nitrogen to the temperature wherein the nitrogen is liquid and suitable for supplying to the feed 32 of circuit 30. As described with reference to fig. 1-4, a heat pump can be applied for this purpose.

Circuit 50 serves to pre-sterilize the circuits where this is necessary. For this purpose the whole apparatus must be completely empty and dry. Bypass line 62 serves to completely dry the environment of the discharge conduit 60 for liquid nitrogen for dosing.

Sterilizing must take place at a temperature of for instance a minimum of 130°C for a number of minutes to be further specified. It will be apparent that the sterilizing time is co-dependent on the design and dimensions of the device.

Before a device is taken into use for the first time a preliminary sterilization takes place. This can take place for a relatively long time, for instance 30 minutes or longer at the above stated temperature of steam or nitrogen of more than 130°C.

After use of the device a renewed sterilization can take place at a temperature of for instance 180-200°C for a relatively short period, for instance 10 seconds.

The aseptic control valve (43) is of known type and can comprise a diaphragm valve. A diaphragm valve is an element which is wholly free of space in which contaminants, particularly micro-organisms, can accumulate.

The whole device according to fig. 5 and also that according to fig. 1-4 can be controlled by a PLC or the like.

## Claims

1. Device for performing a method for eliminating micro-organisms present in a liquified nitrogen, according to which method a first flow (13) of the liquified nitrogen for treating is first heated (11) to above its evaporation temperature into the gas phase, the thus obtained flow of gas is then subjected to a treatment, which treatment comprises filtration and/or sterilization (12), and the treated flow of nitrogen (14) is thereafter cooled by a second flow of liquid nitrogen (10) to below its evaporation temperature into the liquid phase,
**characterized in that**
the device comprises a feed conduit (10) for liquid which branches into a cooling conduit leading to the first circuit (8) of a heat exchanger (9) and a filter conduit (13) leading via a heating device (11) to a filter (12), the outlet of which filter (12) leads to the second circuit (15) of the heat exchanger such that the heating device (11) heats the liquid to above its evaporation temperature into the gas phase and the gas allowed through the filter (12) condenses in the heat exchanger (9),
wherein the outlet of the first circuit (10) is connected via a cooling device (18) to the feed conduit (10), and
wherein the cooling device (18) and the heating device (11) together form part of a heat pump (19).

2. Device as claimed in claim 1, wherein the heat exchanger is of the counterflow type.

## Patentansprüche

1. Vorrichtung zum Durchführen eines Verfahrens zum Eliminieren von Mikroorganismen, die in verflüssigtem Stickstoff vorhanden sind, wobei
gemäß diesem Verfahren ein erster Durchfluss (13) des verflüssigten Stickstoffs zur Behandlung als erstes über seine Dampftemperatur in die Gasphase hinein erhitzt wird (11), wobei
der so erreichte Gasdurchfluss dann einer Behandlung unterzogen wird, wobei
die Behandlung eine Filtration und/oder Sterilisation (12) aufweist, und der Durchfluss von behandeltem Stickstoff (14) danach durch einen zweiten Durchfluss von verflüssigtem Stickstoff (10) unter seine Dampftemperatur in die flüssige Phase hinein gekühlt wird, **dadurch gekennzeichnet, dass**
die Vorrichtung eine Speiseleitung (10) für Flüssigkeit aufweist, die sich in eine Kühlleitung, die zur ersten Leitung (8) eines Wärmetauschers (9) führt und eine Filterleitung (13), die über eine Erhitzungsvorrichtung (11) zu einem Filter (12) führt, verzweigt, wobei
der Auslass dieses Filters (12) zu der zweiten Leitung (15) des Wärmetauschers führt, so dass die Erhitzungsvorrichtung (11) die Flüssigkeit über ihre Dampftemperatur in die Gasphase hinein erhitzt und das Gas, das durch den Filter (12) hindurch gelassen wird, in dem Wärmetauscher (9) kondensiert, wobei
der Auslass der ersten Leitung (10) über eine Kühlvorrichtung (18) mit der Speiseleitung (10) verbunden ist, und wobei
die Kühlvorrichtung (18) und die Erhitzungsvorrichtung (11) zusammen einen Teil einer Wärmepumpe (19) bilden.

2. Vorrichtung gemäß Anspruch 1, wobei der Wärmetauscher vom Gegenstromtyp ist.

## Revendications

1. Dispositif pour réaliser un procédé d'élimination de micro-organismes présents dans l'azote liquéfié, procédé selon lequel un premier flux (13) d'azote liquéfié pour traitement est tout d'abord chauffé (11) à un niveau supérieur à sa température d'évaporation en phase gazeuse, le flux de gaz ainsi obtenu étant ensuite soumis à un traitement, ledit traitement comprenant la filtration et/ou la stérilisation (12) et le flux d'azote traité (14) étant ensuite refroidi par un second flux d'azote liquide (10) jusqu'à un niveau inférieur à sa température d'évaporation en phase liquide,
**caractérisé en ce que**
le dispositif comprend un conduit d'alimentation (10) pour le liquide qui se ramifie en un conduit de refroidissement conduisant au premier circuit (8) d'un échangeur de chaleur (9) et en un conduit de filtrage (13) conduisant via un dispositif de chauffage (11) à un filtre (12), la sortie dudit filtre (12) conduisant au second circuit (15) de l'échangeur de chaleur de sorte que le dispositif de chauffage (11) chauffe le liquide jusqu'à un niveau supérieur à sa température d'évaporation en phase gazeuse et le gaz pouvant passer dans le filtre (12) se condense dans l'échangeur de chaleur (9),
dans lequel la sortie du premier circuit (10) est reliée via un dispositif de refroidissement (18) au conduit d'alimentation (10), et
dans lequel le dispositif de refroidissement (18) et le dispositif de chauffage (11) forment conjointement une partie de la pompe à chaleur (19).

2. Dispositif selon la revendication 1, dans lequel l'échangeur de chaleur est de type à contre-courant.
